Europäisches Patentamt

European Patent Office

Office européen des brevets

(11) Veröffentlichungsnummer: **0 223 161**
**A2**

# EUROPÄISCHE PATENTANMELDUNG

(21) Anmeldenummer: 86115424.3

(22) Anmeldetag: 07.11.86

(51) Int. Cl.⁴: **C12N 1/38** , C12N 1/20 , C12N 1/16 , //C12N1/04

(30) Priorität: 16.11.85 DE 3540721

(43) Veröffentlichungstag der Anmeldung:
27.05.87 Patentblatt 87/22

(84) Benannte Vertragsstaaten:
**BE DE FR GB IT LU NL**

(71) Anmelder: **Lucas Meyer GmbH & Co**
**Ausschläger Elbdeich 62**
**D-2000 Hamburg 28(DE)**

(72) Erfinder: **Ziegelitz, Rüdiger**
**Ausschläger Elbdeich 62**
**D-2000 Hamburg 28(DE)**
Erfinder: **Börs, Heinke-Maria**
**Ausschläger Elbdeich 62**
**D-2000 Hamburg 28(DE)**

(74) Vertreter: **Glaeser, Joachim, Dipl.-Ing. et al**
**Patentanwalt Königstrasse 28**
**D-2000 Hamburg 50(DE)**

(54) **Verfahren zur Aktivierung von in der Lebensmittelverarbeitung verwendeten Mikroorganismen mit Hilfe von Lecithin.**

(57) Verfahren zur Aktivierung von in der Lebensmittelverarbeitung verwendeten Mikroorganismen mit Hilfe von Lecithin.

Starterkulturen für die Lebensmittelindustrie wird bei ihrer Anzucht bzw. Reinkultur dem Nährmedium bei Hefen mit 0,1 -0,7%, bei Bakterien 0,3 -1,0% entöltes Lecithin mit einem Gehalt an acetonunlöslicher Substanz von 97% zugesetzt und das mit Lecithin gut vermischte Nährmedium wird bei 110 -130°C 12 -30 Minuten behandelt und anschließend das mit Lecithin vermischte und sterilisierte Lecithin mit der entsprechenden Kultur beimpft.

EP 0 223 161 A2

## Verfahren zur Aktivierung von in der Lebensmittelverarbeitung verwendeten Mikroorganismen mit Hilfe von Lecithin.

Die Erfindung bezieht sich auf ein Verfahren zur Aktivierung von in der Lebensmittelverarbeitung verwendeten Mikroorganismen mit Hilfe von Lecithin.

Lecithine = Phospholipidgemische kommen in tierischen, pflanzlichen und z. T. in Mikroorganismenzellen vor. Sie sind beteiligt am Zellstoffwechsel. Die Zellen sind in der Lage, Phospholipide selbst zu synthetisieren, sofern die entsprechenden Bausteine von außen zugeführt werden.

Bei der Züchtung von Mikroorganismen, Hefen und Schimmeln werden die Nährmedien entsprechend den Bedürfnissen der jeweiligen Kultur zusammengestellt.

In der klassischen Mikrobiologie wird Soja-oder auch Eilecithin als Inaktivator zur Maskierung für Cetrimid, Chlorhexiden, chlorierte Phenole, Desqualiniumacetat und Polymyxin B eingesetzt, und zwar in Nährmedien wie: Caseinpepton-Lecithin-Polysorbat-Bouillon, AOAC-Letheen Bouillon, Trypticase Soja Agar mit Lecithin·oder Dithion it-Theoglycolat (HS-T) -Bouillon. Lecithin dient hier als "Eiweißfaktor".Die Wirkung als Nährstofflieferant bleibt hierbei unberücksichtigt.

Der Erfindung liegt die Aufgabe zugrunde, den Mikroorganismus außer anderen für ihn wichtigen Makro-und Mikronährstoffen essentielle Bausteine in ausgewogener Zusammensetzung für seinen Synthese-Stoffwechsel zu liefern, die aus dem Lecithin stammen. Hierzu gehören die Phospholipide, die in folgenden Konzentrationen in Reinlecithin vorhanden sind:

Phosphatidylcholin: 20 -23%, Phosphatidylethanolamin: 21 -24%, Phosphatidylinositol: 18-22%, Phosphatidsäure: 5-13% und essentielle Fettsäuren: Linol-Linolensäure.

Damit wird erreicht, daß das Wachstum insbesondere von Mikroorganismen, die in der Lebensmittelindustrie verwendet werden, verbessert wird, die Zellen aktiver sind, die Ausbeute an Kolonien erhöht und die Zellen besser verteilt werden. Dadurch werden die fermentativen Prozesse in den Lebensmitteln beschleunigt, was in der Regel zu höheren Produktausbeuten führt.

Die Erfindung wird anhand der nachfolgenden Beispiele näher erläutert, ohne hierauf beschränkt zu sein:

Beispiel 1

In der milchverarbeitenden Industrie werden vorwiegend als Starterkulturen, Streptococcaceae und Lactobacillaceae verwendet, wie Sc. lactis, Sc. cremoris, Sc. diacetylactis, Sc. thermophilus, Leuc. cremoris, Lb. bulgaricus und Lb.acidophilus. Daneben finden Spezialkulturen in Käsereien Anwendung.

Die Verarbeitung erfolgt nach zwei unterschiedlichen Verfahrensschritten:

a) Die Züchtung der o.g. Starterkulturen erfolgt durch Zusatz von 0,3 -1,0% vorzugsweise 0.5% entöltem Lecithin zum Kulturmedium. Anschließend sterilisiert man je nach Medium bei 110 -130°C und einer Dauer von 12-30 Minuten. Danach wird das Kulturmedium beimpft.

Ergebnis:

Die Zellzahl erhöht sich je nach Starterkultur um den Faktor 10-100. Die Zellen zeigen eine größere Aktivität. Außerdem wird eine evtl. Lyophilisierung der Starterkulturen erleichtert.

Konkrete Anwendung für Lactobazillus bulgaricus

Stammlösung:

100 g Vis-Start-Kulturmedium 10 auf 1000 ml Wasser beimpft mit Lb.bulgaricus, davon je 1 ml auf Standard-I-Nähragar und Standard-I-Nähragar mit 0,1 % entöltem Lecithin. Bebrütung 5 Tage 37°C.

```
Ergebnis Zellzahl:     Standard-I-Nähragar    Standard-I-Nähragar
                       ohne Zusatz            plus 0,1% entöltes
                       332.500                Lecithin
                                              6.525.000
```

(ermittelt als Mittelwert aus 10 Ansätzen).

b) Lecithin wird in sterilisierter Form zusammen mit der Starterkultur der Milch zugesetzt, Dosierung 0,3% -1,0% berechnet auf Milch. Dieses Verfahren läßt sich anwenden auf die Produktion von Joghurt, Kefir, Quark, saurer Sahne und Käse.

Lecithin erhöht auch hier die Aktivität der Zellen, vermehrt die Zellzahl und verbessert entscheidend die Verteilung der Kultur in der Milch, was eine gleichmäßige Struktur von z. B. Joghurt, saurer Sahne und Käse bewirkt.

Beispiel 2

Bei der Brot-und Backwarenherstellung finden u.a. im Sauerteig Lactobacillus brevis L. plantarum und L. fermenti und als weiteres Triebmittel die Hefe Saccharomyces cerevesia Verwendung. Die Sauerteigstarter lassen sich,wie in Beispiel 1 beschrieben, mit entöltem Lecithin als Zusatz zum Nährmedium und sterilisiert zum Sauerteig mit verbessertem Wachstum ziehen.

Bei der Hefe ist ein Zusatz von 0,3 - 0,5 % Lecithin berechnet auf Gesamtnährmedium zu empfehlen parallel zur Melassenährlösung. Das ist auch bei den Sauerteigstartern zu beobachten. Diese Zugabe bewährt sich besonders beim spirituslosen Verfahren.

Trockenhefe behält nach der Trocknung eine höhere "Revitalisierbarkeit" als ohne Zusatz von Lecithin,

Konkrete Anwendung für Saccharomyces cerevesia

Stammlösung:

Physiologische Kochsalzlösung beimpft mit Sm. cerevesia, davon je 1 ml auf Würze-Agar und Würze-Agar mit 0,3% entöltem Lecithin. Bebrütung: 5 Tage 25°C.

```
Ergebnis Zellzahl:     Würze-Agar     Würze-Agar
                       ohne Zusatz    plus 0,3 % entöltes Leci-
                                      thin
                       2.840          91.000
```

(ermittelt als Mittelwert aus 10 Ansätzen )

Beispiel 3

Starterkulturen für die Rohwurstherstellung.

Die für die schnellere Wurstreifung verwendeten Lactobacillen und Aromahefen wie Debaryomyces Hansenii lassen sich ebenfalls mit einem Zusatz von 0,3% -0,8%, vorzugsweise 0,8%, entöltem Lecithin zum Kulturmedium mit einer höheren Ausbeute an Lebendzellzahl und damit größerer Aktivität züchten.

Wichtig bei allen genannten Beispielen ist, daß das Lecithin im Nährmedium gleichmäßig verteilt und anschließend einer thermischen Behandlung von 110-130°C, vorzugsweise 120°C, und einer Dauer von 12-30 Minuten, vorzugsweise 15 Minuten, ausgesetzt wird.

Konkrete Anwendung für Lactobacillus SP L 110

Stammlösung:

Physiologische Kochsalzlösung beimpft mit Lb. SP L 110, davon je 1 ml auf Standard-I-Nähragar und Standard-I-Nähragar mit 0,5 % entöltem Lecithin. Bebrütung 5 Tage 37°C.

```
Ergebnis Zellzahl: Standard-I-Nähragar      Standard-I-Nähragar
                   ohne Zusatz              plus 0,5% entöltes
                                            Lecithin
                   288.700                  5.375.000
(ermittelt als Mittelwert aus 10 Ansätzen)
```

Die Wirkung des Lecithins auf die Mikroorganismen läßt sich nicht bis ins Detail klären. Wahrscheinlich ist aber, daß während des thermischen Prozesses das Lecithin derart hydrolisiert wird, daß Bausteine entstehen, die sozusagen "vorverdaut" sind und daher von den Mikroorganismen für ihre Vermehrung besser genutzt werden können.

Die genannten Beispiele lassen sich auf fast alle in der Lebensmittelindustrie verwendeten Mikroorganismen anwenden.

## Ansprüche

1. Verfahren zur Aktivierung von in der Lebensmittelverarbeitung verwendeten Mikroorganismen mit Hilfe von Lecithin, dadurch gekennzeichnet, daß

a) bei Starterkulturen für die Lebensmittelindustrie bei ihrer Anzucht bzw. Reinkultur dem Nährmedium bei Hefen 0,1 -0,7%, bei Bakterien 0,3 -1,0% entöltes Lecithin mit einem Gehalt an acetonunlöslicher Substanz von 97% zugesetzt werden, und

b) das mit Lecithin gut vermischte Nährmedium bei 110 -130°C, 12 -30 Minuten behandelt wird und anschließend

c) das mit Lecithin vermischte und sterilisierte Lecithin mit der entsprechenden Kultur beimpft wird.

2. Verfahren zur Aktivierung von in der Lebensmittelverarbeitung verwendeten Mikroorganismen mit Hilfe von Lecithin, dadurch gekennzeichnet, daß die Starterkulturen mit sterilem entölten Lecithin mit einem Gehalt an acetonunlöslicher Substanz von ca. 97% dem Lebensmittel in einer Konzentration von 0,3 -1,0% des Lebensmittels zugesetzt werden.